# EUROPEAN PATENT APPLICATION

(11) **EP 3 984 640 A1**
(43) Date of publication of application: **20.04.2022**
(21) Application number: 20202292.7
(22) Date of filing: 16.10.2020
(51) Int. Cl.: B01L 3/00, B82Y 30/00, G01N 33/487

(54) **AN AUTONOMOUS NANOFLUIDIC ANALYSIS DEVICE AND A METHOD FOR THE ANALYSIS OF DNA MOLECULES**

(71) Applicant: Universität Hamburg, 20148 Hamburg (DE)
(72) Inventor: Fernandez-Cuesta, Irene, 22763 Hamburg (DE); Esmek, Franziska, 20357 Hamburg (DE)
(74) Representative: Hauck Patentanwaltspartnerschaft mbB

(57) **Abstract**

A nanofluidic analysis device allowing for spontaneous flow of molecules and method for the analysis of DNA molecules, the device comprising a detection nanochannel, a supply channel in fluid communication with an inlet for the detection nanochannel and a discharge channel in fluid communication with an outlet for the detection nanochannel, so that a fluid comprising DNA molecules introduced into the supply channel may flow along a flow direction through the supply channel, via the inlet into the detection nanochannel, through the detection nanochannel, and out of the outlet into the discharge channel, wherein a laser detector system is provided for analyzing the DNA molecules in the detection nanochannel, wherein both a width and a depth of the inlet and thereby a cross section of the inlet decrease along the flow direction and wherein both a width and a depth of the outlet and thereby a cross section of the outlet increase along the flow direction, wherein the detection nanochannel has a length of not more than 35 µm.

## Description

The invention pertains to a nanofluidic analysis device for the analysis of single DNA molecules as well as to a method for the analysis of DNA molecules via such a nanofluidic analysis device.

For DNA analysis different methods are known which differ in the level of information they provide. DNA sequencing, for example, offers information about the position and composition of every base pair (bp) in the DNA molecule. To reach this high level of information, the DNA molecules are typically broken down into small fragments and are copied, for example using a polymerase chain reaction, to produce millions of these fragments. Subsequently, these fragments are sequenced and the information is reassembled to reconstruct the molecule structure. While DNA sequencing provides a deep level of information, this enormous amount of data also comes at high costs. The sequencing process usually takes days and requires complicated and expensive instrumentation.

If the structural/genomic information of a DNA is sufficient, DNA optical mapping can be used. DNA optical mapping consists of creating a sequence-dependent barcode or fingerprint on the DNA molecules and recording the fluorescent intensity signal of elongated molecules. Typically, in DNA optical mapping a sequence-dependent optical barcode of a DNA molecule is read by imaging them with a sensitive camera coupled to a microscope, wherein the molecules are stretched inside long nanochannels or after combing and immobilizing them on glass substrates. While DNA optical mapping is less costly than DNA sequencing, the instrumentation is still quite expensive, in particular the microscope and the camera. Furthermore, the throughput is relatively low as it relies on imaging and on the interpretation of the data by an expert. To introduce the DNA molecules into the long nanochannels and to stretch the DNA molecules for detection an external force has to be applied, for example via an electrophoresis voltage or a pressure gradient induced by a fluidic pump. This further increases costs and complicates the set up and restricts DNA optical mapping to specialized research laboratories. Furthermore, the maximum length of the molecule which can be visualized at once is limited by the field of view of the camera to around 250 kbp in straight nanochannels. The typical resolution which can be obtained by optical mapping is given by light diffraction, and is typically in the order of 1000 bp.

A device for the on-chip analysis of the length of DNA fragments, obtained by using restriction enzymes, with high throughput is known from US 10,571,428 B2. Said device comprises a reaction nanochannel wherein DNA is fragmented using restriction endonuclease enzymes, the reaction nanochannel merging into a detection nanochannel with an interface between these channels decreasing in diameter. The DNA traverses the relatively long detection nanochannel using electrophoretic forces which are applied via a multitude of electrodes, wherein at one or more locations along the detection nanochannel a signal is detected to map the fragments lengths in the order they occur along the original DNA molecule.

From WO 2016/182811 a fluidic device is known comprising a nanoscale manifold having a plurality of nanoscale elements which are concurrently controlled by a common externally applied pressure or voltage to generate a force gradient within a transport nanochannel.

From US 9,447,451 a nanofluidic device with 2D inlets and outlets is known, wherein a width of an inlet leading to a detection nanochannel decreases and a width of an outlet increases along the flow direction of molecules.

It is an object of the invention to provide a nanofluidic analysis device and a method that allow for simpler, less expensive analysis of DNA, which at the same time provides more information than previous works. It is a further object of the invention to allow for a high throughput of DNA molecules and a real-time readout of the finrgerpint of the labelled molecules.

The invention solves these objects with a nanofluidic analysis device of claim 1 and a method of claim 11. Advantageous embodiments are disclosed in the dependent claims, the description as well as the figures.

A nanofluidic analysis device for the analysis of DNA molecules according to the invention comprises a detection nanochannel, a supply channel in fluid communication with an inlet for the detection nanochannel and a discharge channel in fluid communication with an outlet for the detection nanochannel, so that a fluid comprising DNA molecules introduced into the supply channel may flow along a flow direction through the supply channel, via the inlet into the detection nanochannel, through the detection nanochannel, and out of the outlet out of the detection nanochannel into the discharge channel, wherein a laser detector system is provided for analyzing the DNA molecules in the detection nanochannel, wherein both a width and depth of the inlet and thereby a cross section of the inlet decrease along the flow direction and wherein both a width and depth of the outlet and thereby a cross section of the outlet increase along the flow direction, wherein the detection nanochannel has a length of not more than 35 µm.

The invention further pertains to a method for the analysis of DNA molecules via a nanofluidic analysis device, wherein a fluid comprising DNA molecules is introduced into a supply channel of the nanofluidic analysis device, the fluid spontaneously flowing along a flow direction through the supply channel, into a detection nanochannel of the nanofluidic analysis device via an inlet, both a width and depth of the inlet and thereby a cross section of the inlet decreasing along the flow direction, the detection nanochannel having a length of not more than 35 µm, the fluid having entered the detection nanochannel spontaneously flowing along the flow direction through the detection nanochannel, wherein the DNA molecules inside the detection nanochannel are analyzed via a laser detector system, the fluid further spontaneously leaving the detection nanochannel along the flow direction through an outlet into a discharge channel, both a width and depth of the outlet and thereby a cross section of the outlet increasing along the flow direction.

The nanofluidic analysis device according to the invention is able to execute the inventive method. Accordingly, the method steps of the inventive method may be executed by the inventive nanofluidic analysis device. Explanations made with respect to the device apply to the inventive method mutatis mutandis, explanations made with respect to the inventive method applied to the inventive device mutatis mutandis.

With the nanofluidic analysis device according to the invention, DNA molecules may be analyzed, may in particular be detected via the laser detector system. The laser detector system may comprise an excitation and photo detector. The analysis, which in particular allows obtaining the fluorescent barcode of each molecule, may comprise identifying a species associated with the respective DNA molecule. Such DNA analysis has applications in medicine, in liquid biopsy, forensics, paleontology, evolutionary sciences, control of animal and vegetable species, food safety traceability and authenticity and many more. DNA analysis can, for example, be used to identify individuals and suspects in a crime scene, control the origin and composition of processed food and, probably most relevant, can be used in the medical sector. For example, analyzing the DNA of a bacteria strain can tell in advance about its resistances to antibiotics, DNA and RNA molecules and fragments circulating in the blood can give information about viral infections which might have with disease screening of infected people in case of an epidemic outbreak, even at early stages. The inventive device and method can in particular be used to identify and discriminate pathogens. The inventive device and method are particularly suited for such DNA analysis as they allow for cheap and fast analysis, as will be explained in the following.

According to the invention, a fluid comprising DNA molecules to be analyzed is transported from the supply channel into the detection nanochannel and out of the detection nanochannel into a discharge channel. The fluid comprising the DNA molecules flows through these channels along a flow direction, wherein the DNA molecules flowing along the detection nanochannel pass a laser spot projected by the laser detector system, the laser spot exciting the fluorescence of the molecules, and a photodetector of the laser detector system, which detects the emitted fluorescence in real time, and can be used to analyze the DNA. The inventive device comprises an inlet which established a connection between the detection nanochannel and the supply channel and an outlet which establishes a connection between the detection nanochannel and the discharge channel.

According to the invention, the inlet has a cross-section that decreases along the flow direction and the outlet has a cross-section which increases along the flow direction of the fluid comprising the DNA molecules. The cross-section meaning, in particular, an area perpendicular to the flow direction. In particular, a width and a depth of the inlet decrease along the flow direction, the flow direction being parallel to a length of the inlet. Correspondingly, a width and a depth of the outlet increase along the flow direction, that is parallel to a length of the outlet. The width and depth are the extensions of the inlet/outlet in a plane perpendicular to the length of the inlet/outlet and therefore perpendicular to the flow direction. The increase or decrease in cross-section can be continuous or stepped as will be explained later. In particular, the inlet to the detection nanochannel may be tapered, while the outlet out of the detection nanochannel may be widening with respect to the flow direction. Thus, the outlet may be considered to be tapered along a direction contrary to the flow direction. In particular the cross-section of the inlet may decrease linearly along the flow direction and/or the cross-section of the outlet may increase linearly along the flow direction. However, the cross-section may increase or decrease in other ways, e.g. exponentially. With the width and depth both increasing or decreasing as explained above three-dimensional inlets or outlets are realized which will also be called 3D inlets/outlets in the following. The inlet and/or outlet may have a conical form. Furthermore, the inventive detection nanochannel has a length of no more than 35 µm.

The inventors realized that such a detection nanochannel facilitates the flow of the fluid comprising the DNA molecules through the detection nanochannel. Thanks to the 3D inlet, the 3D outlet as well as the relatively short length of the detection nanochannel of the inventive device the flow of fluid comprising the DNA molecules along the flow direction is facilitated. Actually, as the inventors realized, no external force is needed to move the fluid through the detection nanochannel and to stretch the DNA molecules in the nanochannels. Thus, with the invention it is not necessary to provide for electrophoresis via applying an electric field or to provide for an active flow of the fluid via a pump pumping the fluid through the detection nanochannel. On the contrary, the fluid and the molecules spontaneously flow through the inlet into the detection nanochannel and through the outlet out of the detection nanochannel. The fluid spontaneously flowing means that the fluid flows in a self-acting manner or on itself, in particular without the appliance of an external force. Such a spontaneous flow is possible due to the 3D inlet and the 3D outlet of the detection nanochannel as well as the relatively small length of the detection nanochannel, relatively small meaning a length of no more than 35 µm. The spontaneous flow is probably an effect of diffusion, ionic, and electroosmotic forces acting on the fluid, given partially by the charged walls of the polymer nanochannel. While such forces may also be present in nanochannels of greater length and without tapered inlets or widening outlets, for these devices the forces are too weak to allow for the explained autonomous flow-through of the fluid through the nanochannel. This is why the nanofluidic analysis devices according to the prior art always need an external force, for example, electrophoresis, to achieve a sufficient flow of fluid comprising DNA through the nanochannel. The device according to the invention, however, thanks to the inventive design of the nanochannel allows for an autonomous flow-through analysis of DNA molecules at high speeds, in particular at real-time, and therefore with a high throughput without the need of external forces. In particular, it is possible with the invention to obtain a fingerprint (barcode) of the DNA single molecules, which spontaneously enter the nanochannel, and where they are stretched for read-out. This method allows for a simpler, less expensive analysis of DNA in a device with autonomous flow.

Thus, the inventive method may comprise that the fluid comprising the DNA molecules moves along the flow direction without an external force applied, in particular without a voltage difference or a pressure difference being applied to the inlet. Thus, spontaneous flow is achieved without applying an electric field and/or an active flow via a pump. The nanofluidic analysis device, in particular the detection nanochannel, according to the invention, may therefore be adapted such that the fluid comprising the DNA molecules may flow along the flow direction without an external force needing to be applied, in particular without the need for an electric field and/or without the need for an active flow via a pump.

The inventive device and method have several advantages over DNA sequencing. For example, less DNA material is needed. Furthermore, the analysis is much faster and simpler. In particular, with the invention, an analysis in real-time is possible and less expensive equipment is needed. There is no loss of structural information and many different samples can be analyzed in a reasonable time. In addition, a signal provided by the laser detector system is straightforward to read and interpret, unlike the enormous bioinformatic effort needed for assembling the reads from the sequencing. Compared to microscope-based optical mapping the inventive device and method are also distinctively faster and therefore allow for higher throughput. In particular, barcodes may be read out at a higher resolution with the inventive method and device thanks to the laser detector system; a resolution of 800 bp having been achieved in preliminary results. In addition, the device according to the invention is cheaper, faster, and easier to produce. No expensive microscope or camera are needed. On the one hand, signals from very short DNA fragments (few tens or hundreds of base pairs long) may be detected and discriminated, while on the other hand the structural configuration of unfragmented ultralong molecules (mega base pair long) may be read as well without limitations in length; and also molecules with intermediate length. Thus, with the inventive device and method, the barcode of a DNA molecule may be read out in particular autonomously in real-time with high throughput.

According to an embodiment the width of the inlet decreases uniformly or in multiple gradual steps along the flow direction. According to a respective embodiment the depth of the inlet decreases uniformly or in multiple gradual steps along the flow direction. Thus, the cross-section of the inlet may decrease uniformly or in multiple gradual steps along the flow direction. The outlet may be designed accordingly. Thus, the width of the outlet may increase uniformly or in multiple gradual steps along the flow direction. Also, the depth of the outlet may increase uniformly or in multiple gradual steps along the flow direction. Accordingly, the cross-section of the outlet may increase uniformly or in gradual steps. Increasing or decreasing uniformly may be understood as increasing or decreasing continuously, in particular without any steps. For example, the inlet and/or outlet may have a conical form. Increasing or decreasing in multiple gradual steps may be understood as a sequence of small steps, the steps being small in particular with respect to a largest width and/or depth of the inlet/outlet. Also, combinations of these embodiments are possible. For example, the width of the inlet may decrease uniformly while the depth of the inlet decreases in multiple gradual steps or the other way around. The same may apply mutatis mutandis to the outlet. For example, the width of the outlet may increase uniformly while the depth of the outlet increases in multiple gradual steps or the other way around.

Furthermore, the inlet and outlet may be symmetric or asymmetric. According to a respective embodiment the inlet and the outlet are of a symmetric or an asymmetric configuration. In particular, the whole arrangement of the detection nanochannel with the inlet on the one end and the outlet on the other end may be of a symmetric or an asymmetric configuration. A symmetric configuration means, in particular, a mirror symmetry around an axis perpendicular to the length of the detection nanochannel or inlet or outlet.

According to an embodiment pillars can be arranged in the inlet and/or in the outlet. These pillars may be arranged on an inner side wall of the inlet and may extend into the inlet in a direction perpendicular to the flow direction, in particular perpendicular to the length of the inlet and thus along the width and/or the depth of the inlet. Accordingly, the pillars may be arranged on an inner side wall of the outlet and may extend into the outlet in a direction perpendicular to the flow direction, in particular perpendicular to the length of the outlet and thus along the width and/or the depth of the outlet. The pillars may extend from a first side wall to a second side wall opposite the first side wall along the whole width or depth of the inlet or outlet. Such pillars may further facilitate the analysis of the DNA molecules. In particular, such pillars may facilitate the stretching of the molecules and slow them down.

As mentioned, the length of the detection nanochannel is not exceeding 35 µm. In an embodiment, the detection nanochannel has a length of no more than 30 µm, in particular no more than 25 µm. As mentioned above, nanochannels longer than 35 µm may not allow for a sufficient flow of fluid through the detection nanochannel without an external force when the length of the molecules is in the same range. A length of 30 µm or less may facilitate the spontaneous flow of fluid comprising DNA molecules through the detection channel even further. The length of the detection channel should, of course, be large enough to allow for reliable detection of the DNA molecules via the laser detector system. Thus, the detection nanochannel in an embodiment has a length of at least 1 µm. The detection nanochannel may have a length in the range of 1 µm to 35 µm, in particular of 5 µm to 30 µm, in a further embodiment of 5 µm to 25 µm.

In addition to the 3D inlets and outlets and to the length of the detection nanochannel the cross-section of the detection nanochannel itself may also be a relevant factor for allowing the inventive spontaneous flow as the inventors found out. In particular, it is helpful for the detection nanochannel to have a not too large cross-section in order to allow for optimal stretching of the DNA molecules inside the nanochannels, and to obtain the barcode (fingerprint) with better resolution. But, the smaller the cross-section, the more difficult it is to obtain a flow of DNA molecules, and even more difficult to get sufficient flow in a spontaneous manner. The smaller the cross-section, the more important it is having a proper inlet and outlet. Furthermore, a too large cross-section of the nanochannel might lead to coiling of the DNA molecules and formation of hairpins, and even clogging at the entrance of the nanochannel. According to an embodiment, the detection nanochannel has a cross-section of no more than 62,500 nm² (sixty two thousand five hundred), in particular the cross-section may be in a range between 62,500 nm² and 10,000 nm² (ten thousand). Thus, if the detection nanochannel has a square cross-section, the width and the depth of the detection nanochannel may in particular be no more than 250 nm. In particular, width and depth may be between 250 nm and 100 nm each for optimal work. If the detection nanochannel has a circular cross-section, the diameter of the cross-section is preferably no more than 283 nm, in particular preferably in the range between 113 and 283 nm.

According to an embodiment the detection nanochannel is made of a polymer. The supply channel and/or the discharge channel may be made of the same polymer. The channels may be produced via nano imprint lithography. The channels being made of such a polymer, in particular via nano imprint lithography, allows for a faster, cheaper and easier production of the device.

According to an embodiment, the laser detector system comprises a laser source for illuminating the DNA molecules inside the detection nanochannel and a detecting element, in particular a photon counter, for detecting fluorescent light emitted by the illuminated DNA molecules. Thus, the DNA molecules inside the detection nanochannel may be illuminated via the laser detector system and a corresponding signal of fluorescent light emitted from the DNA molecules may be recorded as a fluorescence signal. Analyzing the DNA molecules may comprise the recording of the fluorescence signal as well as retrieving a barcode of the DNA molecule in real-time. The nanofluidic analysis device may accordingly be adapted to record a fluorescence signal of the DNA molecule and to retrieve a barcode of the DNA molecule in real-time. The nanofluidic analysis device may comprise a control unit, which may be adapted to execute these steps. The control unit and/or the nanofluidic analysis device may further be adapted to compare the retrieve barcode of the DNA molecule to theoretical barcodes. Thus, the method may comprise a step of comparing the retrieved barcode of the DNA molecule to theoretical barcodes. The theoretical barcodes may be part of a reference library of DNA sections or sequences which may be used to uniquely identify an organism to a species. Thus, with such a DNA barcoding the inventive method and device allow for a species identification as mentioned above. In particular, pathogens may be identified. Thanks to the inventive device and method such an identification can be done continuously at high speeds basically in real time. The control unit may be programmed for an automatic detection and analysis.

Before adding the fluid comprising the DNA molecules into the supply channel, the DNA may be barcoded. According to an embodiment of the invention, the method comprises the step of barcoding the DNA molecules via competitive binding to differentiate between AT-rich regions and GC-rich regions of the DNA molecule. Depending on the agent used either the AT-rich regions or the GC-rich regions of the DNA molecule are highlighted in the fluorescence signal retrieved by the laser detector system. These highlighted regions may make up the lines of the barcode. This barcode may then, as explained, be compared to a theoretical barcode to identify the species. As agents, for example, Netropsin or Actinomycin D may be used. Further, an intercalating dye, as for example TOTO-3, may be used. Another method for barcoding the DNA, in particular, for marking specific sites, genes or regions of interest within the molecule might be used.

According to a further embodiment of the method, the fluid comprises a buffer medium. The fluid comprising the DNA molecules may thus also comprise a buffer medium, the buffer medium further facilitating the flow of the fluid through the channels. Such a buffer medium may reduce adhesion forces of the DNA molecules to the inner surfaces of the channels. As a buffer medium for example TRIS-Borat-EDTA (TBE) may be used. For example, the DNA molecules to be analyzed may be diluted in a degassed TBE aqueous buffer.

The invention also pertains to the use of a device as described above, wherein a fluid comprising DNA molecules is introduced into the supply channel and flows spontaneously along the flow direction through the supply channel without the help of an external force, in particular without a voltage difference or a pressure difference being applied to the inlet and outlet.

Embodiments of the invention will in the following be explained with respect to the figures.
- Fig. 1: shows a nanofluidic analysis device according to the invention in a perspective view,
- Fig. 2: shows different designs for a detection nanochannel,
- Fig. 3: shows a real-time readout of the laser detector system as well as a comparison of two different DNA molecule barcodes to theoretical barcodes, and
- Fig. 4: shows four measurements of DNA molecules of the same type compared to a theoretical barcode, the DNA molecules having been barcoded with different agents.

In the following the same reference signs pertain to the same elements unless stated otherwise.

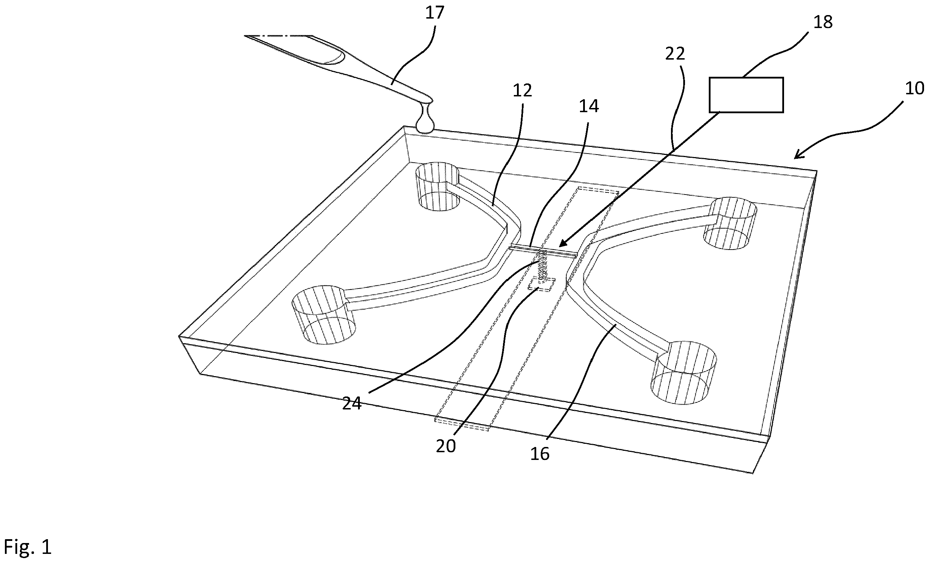

In Fig. 1 a nanofluidic analysis device according to the invention is shown. The device comprises a detection nanochannel 14 which is in fluid communication with a supply channel 12 and a discharge channel 16. A fluid comprising DNA molecules may be introduced into the supply channel 12 as is indicated by a pipette 17 dropping fluid. The fluid introduced into the supply channel 12 first wets the fluid system by capillary forces, and then the molecules flow spontaneously without the need of an external force along a flow direction F through an inlet of the detection nanochannel 14 into the detection nanochannel 14, through the detection nanochannel 14, and out of an outlet of the detection nanochannel 14 into the discharge channel 16.

While passing the detection nanochannel 14, the DNA molecules inside the fluid are analyzed via a laser detector system 18, 20. The laser detector system comprises a laser source 18 illuminating the DNA molecules via a laser beam 22, and a photon counter 20 as a detecting element detecting fluorescent light emitted by the illuminated DNA molecules, the fluorescent light being indicated in Fig. 1 at reference sign 24. This way, a barcode of the DNA molecules may be read out as will be explained later. The device 10 may be produced via nanoimprint lithography and may be made of a polymer. Such a nanolithography process is of low cost and quick manufacture.

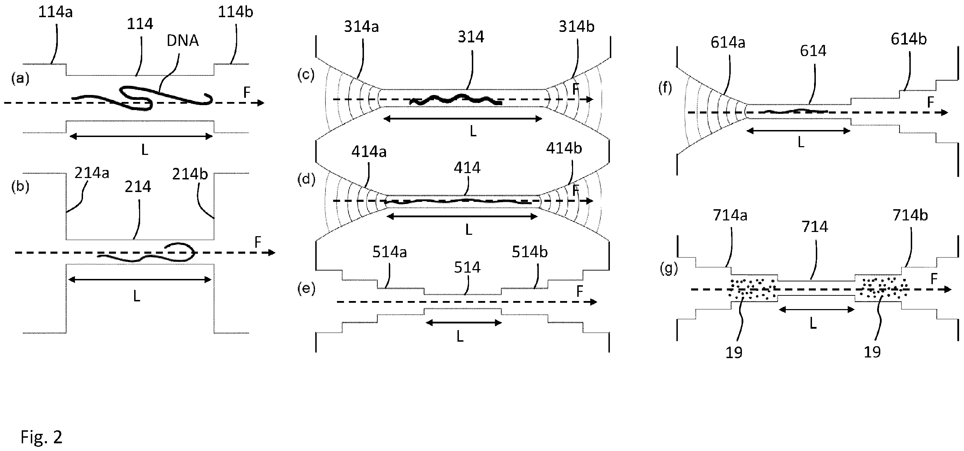

Different designs of detection nanochannels are shown in Fig. 2. While in Figs. 2a and 2b non-claimed nanochannels are shown, Figs. 2c to 2g show nanochannels according to the invention. A detection nanochannel 114 has a large width and therefore a large cross-section. The nanochannel 114 comprises an inlet 114a and an outlet 114b which are larger in cross-section than the nanochannel 114. With respect to a flow direction F, the cross-section is reduced abruptly by a relatively large amount when transitioning from the inlet 114a to the channel 114 and increased abruptly by a relatively large amount when transitioning from the channel 114 to the outlet 114b as there is a large step-like transition. Such a nanochannel design may lead to coiling of the DNA, as can be seen in Fig. 2a. This is due, in particular to the nanochannel being too wide or too deep. In Fig. 2b a further non-claimed embodiment is shown, wherein a nanochannel 214 of a smaller width is shown. Again, there is a step-like, even more abrupt transition between the inlet 214a and the channel 214 as well as between the channel 214 and the outlet 214b. While the width of the nanochannel is small enough to minimize the coiling of the DNA, the DNA may form so-called hairpins, wherein a front end of the DNA strand is folded as shown in Fig. 2b. Such hairpins are primarily caused due to the abrupt changes in cross-section.

Accordingly, the inventors derived detection channels as shown in Figs. 2c to 2g.

As can be seen in Fig. 2c, a cross-section of an inlet 314a leading to a detection nanochannel 314 decreases continuously along the flow direction F, wherein a cross-section of an outlet 214b increases continuously along the flow direction F. This way, the inlet 314a is tapered with respect to the flow direction and the outlet 314b is widening with respect to the flow direction. Thus, in a direction opposite to the flow direction F, the outlet 314b is tapered as well. The cross-section of the inlet 314a decreases as both a width as well as a depth of the inlet 314a decrease along the flow direction F. In Figures 2 the width of the inlet and outlet denotes an extension in the image plane perpendicular to the length L and the flow direction F, while the depth denotes an extension perpendicular to a length of inlet/outlet (and therefore the length L of the nanochannel) and the flow direction F as well as perpendicular to the image plane. The cross-section of the outlet 314b increases as both a width as well as a depth of the outlet 314b increase along the flow direction F. With the width and depth both increasing or decreasing 3D inlets/outlets are realized. Furthermore, the detection nanochannel 14 is relatively narrow, having a cross-section of no more than 62 500 nm². Additionally, a length L of the nanochannel 14 is no more than 35 µm. Such 3D inlets and the relatively short length of the detection nanochannel lead to spontaneous flow of DNA molecules as well as to a stretching of the molecules as will be explained later on.

The embodiment in Fig. 2d showing an inlet 414a, a detection nanochannel 414 and an outlet 414b, differs from the embodiment of Fig. 2c in that the cross-section of the detection nanochannel 414 is smaller than that of the detection nanochannel 314. As can be seen in Figs. 2c and 2d such configurations of the detection nanochannel allow for an optimally stretched DNA strand which may be analyzed reliably, wherein due the smaller cross-section of the detection nanochannel 414 the DNA molecule is stretched even further than in the detection nanochannel 314. While in these embodiments the inlet and outlet are of a conical form, other forms of inlets and outlets increasing or decreasing are also covered by the invention. For example, the increase or decrease in cross-section may not be a linear one or may be realized by multiple steps as shown in figures 2e to 2g.

In Fig. 2e both the width and depth of an inlet 514a leading to a detection nanochannel 514 decrease in multiple gradual steps along the flow direction F. Accordingly, both the width and depth of an outlet 514b increase in multiple gradual steps along the flow direction F. In contrast to the arrangements shown in Figs. 2a, 2b there are no abrupt steps, but rather small subsequent steps which reduce or increase the cross-section of the inlet 514a or outlet 514b gradually. In this manner 3D inlets and outlets are realized as well.

While the configuration of the inlet, detection nanochannel and outlet is symmetrical in Figs. 2c to 2e, it may also be asymmetrical as is shown in Fig. 2f. In the embodiment in Fig. 2f the width and depth and therefore the cross-section of an inlet 614a decreases uniformly along the flow direction F, while the width and depth and therefore the cross-section of an outlet 614b increases in multiple gradual steps.

In Fig. 2g an embodiment is shown, wherein an inlet 714a and an outlet 714b each comprise a multitude of pillars extending from one side wall of the inlet 714a and the outlet 714b along the depth into the inlet and outlet, respectively. Such pillars may further facilitate the spontaneous flow and stretching of the DNA molecules.

As the inventors found out, the inventive designs of the detection nanochannel allow for a flow of the fluid comprising the DNA molecules through the analysis device without the need of an external force. While prior art devices always use some kind of external force to force the fluid through the nanochannel, for example via electrophoresis applied via electrodes or via a normal or hydraulic pump producing a flow, the device according to the invention does not need such an external force as the fluid and the DNA molecules in it flow spontaneously into and through the detection nanochannel. This is probably due to enhanced diffusion, ionic, electroosmotic and capillary forces, which are the result of the specific, inventive form of the detection nanochannel and its inlets and outlets. In particular, the length of the detection nanochannel being not more than 35 µm and the 3D inlet as well as the 3D outlet allow for a spontaneous flow without the need of external force. The nanofluidic analysis device according to the invention is therefore way simpler and less costly than prior art devices. In addition, producing the multilevel, multidimensional fluidic devices by imprinting simplifies the fabrication process. With the inventive device DNA molecules may be analyzed continuously basically in real time, in particular via barcoding as will be explained in the following.

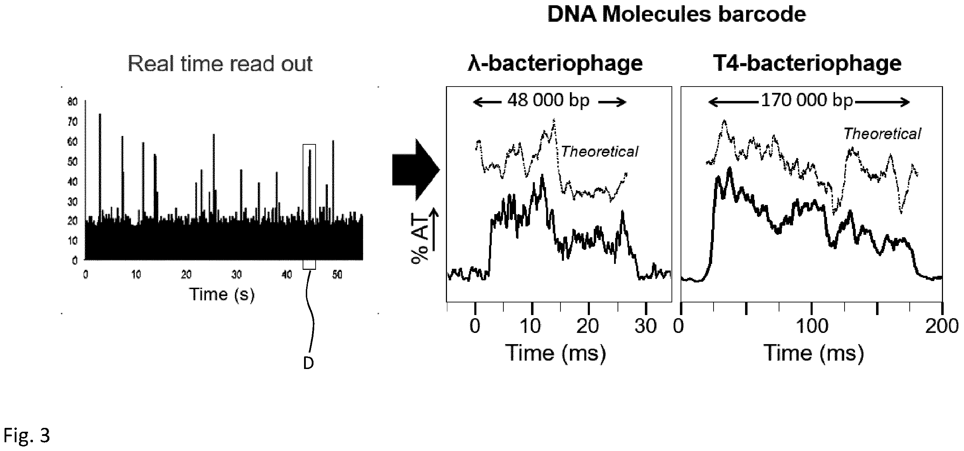

In Fig. 3 a real-time readout of the signal produced by the photon counter 20 of the device 10 is shown at the left. This diagram shows an intensity over the time in seconds. Each of the peaks that can be seen in this diagram correspond to one DNA molecule. The signal is a fluorescence signal, the peaks corresponding to an amount of light emitted by the DNA molecules via fluorescence. Looking at a peak marked as detail D in this diagram may give one of the diagrams on the right. On the right two fingerprints or barcodes of two different bacteriophages are shown, a signal f1 of a lambda-bacteriophage and a signal f2 of a T4-bacteriophage. The peak of the f1-signal has a width of approx. 48 000 base pairs. The f2 signal has a width of approx. 170 000 base pairs. The f1 and f2 signals, which are the lower ones in each of the diagrams, are compared each to respective theoretical signals shown in the diagrams above the f1 and f2 signals. Via such a comparison the respective bacteriophage can be identified. Thus, with this method it can be discriminated between different pathogens.

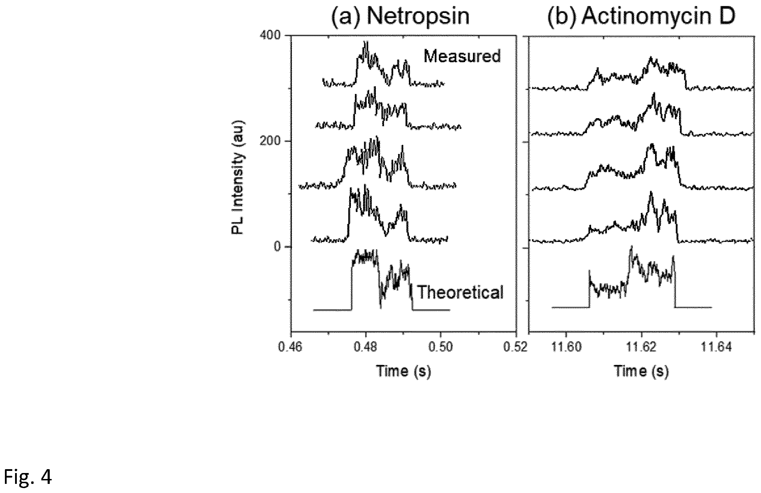

The DNA molecules shown in Fig. 3 have been barcoded via competitive binding. Competitive binding can be achieved via different agents. The DNA molecules can, for example, be barcoded using Netropsin and TOTO-3 which highlight the GC-rich areas in the signals, leaving the AT-rich areas dark. This can be seen in Fig. 4a. Here measurements of four molecules and a theoretical signal at the bottom are shown. Alternatively, the DNA molecules may be barcoded for example with Actinomycin D and TOTO-3. With this chemical a complementary signal to that from Netropsin may be achieved, where AT-rich areas highlighted, leaving the GC-rich areas dark as can be seen in Fig. 4b.

### List of reference numbers

- 10: nanofluidic analysis device
- 12: supply channel
- 14: detection nanochannel
- 16: discharge channel
- 17: pipette
- 18: laser source
- 20: photon counter
- 22: laser beam
- 24: flourescent light
- 114-714: detection nanochannels
- 114a-714a: inlets
- 114b-714b: outlets
- F: flow direction
- L: length

## Claims

1. A nanofluidic analysis device (10) for the analysis of DNA molecules, comprising a detection nanochannel (14, 314, 414, 514, 614, 714), a supply channel (12) in fluid communication with an inlet (314a, 414a, 514a, 614a, 714a) for the detection nanochannel (14, 314, 414, 514, 614, 714) and a discharge channel (16) in fluid communication with an outlet (314b, 414b, 514b, 614b, 714b) for the detection nanochannel (14, 314, 414, 514, 614, 714), so that a fluid comprising DNA molecules introduced into the supply channel (12) may flow along a flow direction (F) through the supply channel (12), via the inlet (314a, 414a, 514a, 614a, 714a) into the detection nanochannel (14, 314, 414, 514, 614, 714), through the detection nanochannel (14, 314, 414, 514, 614, 714), and out of the outlet (314b, 414b, 514b, 614b, 714b) out of the detection nanochannel (14, 314, 414, 514, 614, 714) into the discharge channel (16), wherein a laser detector system (18, 20) is provided for analyzing the DNA molecules in the detection nanochannel (14, 314, 414, 514, 614, 714), wherein both a width and a depth of the inlet (314a, 414a, 514a, 614a, 714a) and thereby a cross section of the inlet (314a, 414a, 514a, 614a, 714a) decrease along the flow direction (F) and wherein both a width and a depth of the outlet (314b, 414b, 514b, 614b, 714b) and thereby a cross section of the outlet (314b, 414b, 514b, 614b, 714b) increase along the flow direction (F), wherein the detection nanochannel (14, 314, 414, 514, 614, 714) has a length of not more than 35 µm.

2. The device of claim 1, wherein the width of the inlet (314a, 414a, 514a, 614a, 714a) decreases uniformly or in multiple gradual steps along the flow direction (F) and/or wherein the depth of the inlet (314a, 414a, 514a, 614a, 714a) decreases uniformly or in multiple gradual steps along the flow direction (F) and/or wherein the width of the outlet (314b, 414b, 514b, 614b, 714b) increases uniformly or in multiple gradual steps along the flow direction (F) and/or wherein the depth of the outlet (314b, 414b, 514b, 614b, 714b) increases uniformly or in multiple gradual steps along the flow direction (F).

3. The device of claim 1 or 2, wherein the inlet (314a, 414a, 514a, 614a, 714a) and the outlet (314b, 414b, 514b, 614b, 714b) are of a symmetric or an asymmetric configuration.

4. The device of one of the preceding claims, wherein pillars (19) are arranged in the inlet (714a) and/or in the outlet (714b).

5. The device of one of the preceding claims, wherein the detection nanochannel (14, 314, 414, 514, 614, 714) has a length of at least 1 µm.

6. The device of one of the preceding claims, wherein the detection nanochannel (14, 314, 414, 514, 614, 714) has a cross section of not more than 62,500 nm², in particular between 62,500 nm² and 10,000 nm².

7. The device of one of the preceding claims, wherein the laser detector system (18, 20) comprises a laser source (18) for illuminating the DNA molecules inside the detection nanochannel (14, 314, 414, 514, 614, 714) and a detecting element (20), in particular a photon counter, for detecting fluorescent light emitted by the illuminated DNA molecules.

8. The device of one of the preceding claims, wherein the nanofluidic analysis device, in particular a control unit of the device, is adapted to record a fluorescence signal of the DNA molecule, and to retrieve a barcode of the DNA molecule in real-time.

9. The device of claim 8, wherein the nanofluidic analysis device, in particular the control unit of the device, is adapted to compare the retrieved barcode of the DNA molecule to theoretical barcodes.

10. Use of a device according to one of the preceding claims, wherein a fluid comprising DNA molecules is introduced into the supply channel (12) and flows spontaneously along the flow direction (F) through the supply channel (12) without the help of an external force, in particular without a voltage difference or a pressure difference being applied to the inlet and outlet.

11. Method for the analysis of DNA molecules via a nanofluidic analysis device, wherein a fluid comprising DNA molecules is introduced into a supply channel (12) of the nanofluidic analysis device, the fluid spontaneously flowing along a flow direction (F) through the supply channel (12), into a detection nanochannel (14, 314, 414, 514, 614, 714) of the nanofluidic analysis device via an inlet (314a, 414a, 514a, 614a, 714a), both a width and a depth of the inlet (314a, 414a, 514a, 614a, 714a) and thereby a cross section of the inlet (314a, 414a, 514a, 614a, 714a) decreasing along the flow direction (F), the detection nanochannel (14, 314, 414, 514, 614, 714) having a length of not more than 35 µm, the fluid having entered the detection nanochannel (14, 314, 414, 514, 614, 714) spontaneously flowing along the flow direction (F) through the detection nanochannel (14, 314, 414, 514, 614, 714), wherein the DNA molecules inside the detection nanochannel (14, 314, 414, 514, 614, 714) are analyzed via a laser detector system (18, 20), the fluid further spontaneously leaving the detection nanochannel (14, 314, 414, 514, 614, 714) along the flow direction (F) through an outlet (314b, 414b, 514b, 614b, 714b) into a discharge channel (16), both a width and a depth of the outlet (314b, 414b, 514b, 614b, 714b) and thereby a cross section of the outlet (314b, 414b, 514b, 614b, 714b) increasing along the flow direction (F).

12. Method of claim 11, wherein the fluid comprising the DNA molecules moves along the flow direction (F) without an external force, in particular without a voltage difference or a pressure difference being applied to the inlet (314a, 414a, 514a, 614a, 714a) and outlet (314b, 414b, 514b, 614b, 714b).

13. Method of claim 12, wherein analyzing the DNA molecules inside the detection nanochannel (14, 314, 414, 514, 614, 714) via the laser detector system (18, 20) comprises recording a fluorescence signal of the DNA molecule and retrieving a barcode or fingerprint of the DNA molecule in real-time.

14. Method of claim 13, **characterized by** comparing the retrieved barcode or fingerprint of the DNA molecule to one or more theoretical barcodes or fingerprints.

15. Method of one of claims 11 to 14, **characterized by** barcoding the DNA molecules before introduction into the supply channel (12) via competitive binding to differentiate between AT-rich regions and GC-rich regions of the DNA molecule, or by using fluorophores to selectively mark specific sites, genes or regions of interest within the DNA molecule.
